# EUROPÄISCHE PATENTSCHRIFT

(11) **EP 0 334 177 B1**
(45) Veröffentlichungstag und Bekanntmachung des Hinweises auf die Patenterteilung: **07.06.1995**
(21) Anmeldenummer: 89104529.6
(22) Anmeldetag: 14.03.1989
(51) Int. Cl.: A61K 33/26, A61K 33/14

(54) **Oral verabreichbares pharmazeutisches Mittel, insbesondere zur Eisen- und Magnesiumsubstitutionstherapie**
Pharmaceutical product for oral administration, particularly for the iron and magnesium substitution therapy
Produit pharmaceutique pour administration orale, en particulier pour la thérapie de substitution du fer et du magnésium

(30) Priorität: 22.03.1988 DE 3809625
(43) Veröffentlichungstag der Anmeldung: 27.09.1989
(73) Patentinhaber: Verla-Pharm Arzneimittelfabrik Apotheker H.J. von Ehrlich GmbH & Co. KG, D-82327 Tutzing (DE)
(72) Erfinder: Helbig, Joachim Dr., D-8132 Tutzing (DE); Classen, Hans Georg Dr., D-7000 Stuttgart 70 (DE)
(74) Vertreter: Müller-Boré & Partner Patentanwälte

(56) Entgegenhaltungen:
- EP-A- 0 061 175
- CH-A- 294 891
- ROTE LISTE 1961, Editio Cantor, Aulendorf/Württ, DE, Gevrabon-Saft "Lederle"
- Deutsche Gesellschaft für Ernährung: Empfehlungen für die Nährstoffzufuhr, 4. Auflage , 1985, Seiten 34, 35, 3726-29, 43
- Allgemeine und spezielle Pharmakologie und Toxicologie, 5. Auflage, Seiten 338-339

## Beschreibung

Die Erfindung betrifft die Verwendung einer bzw. mehrerer pharmazeutisch und physiologisch verträglicher Eisenverbindung(en) und einer bzw. mehrerer pharmazeutisch und physiologisch verträglicher Magnesiumverbindung(en) zur Herstellung eines pharmazeutischen Mittels zur Eisen- und Magnesiumsubstitutionstherapie.

Durch die heutige Ernährung wird der menschliche Organismus mit wesentlichen Elementen, wie Magnesium, Eisen oder Kalzium, unterversorgt. Ein Magnesium- und Eisenmangel macht sich insbesondere bei schwangeren Frauen bemerkbar, die einen erhöhten Magnesiun- und Eisenbedarf haben, um insbesondere Anämien und schlechten Geburtsausbeuten vorzubeugen (vgl. K. Huth und R. Kluthe, "Lehrbuch der Ernährungstherapie", Georg Thieme Verlag Stuttgart-New York, S. 100 - 101; S. Koller, "Risikofaktoren der Schwangerschaft", Springer Verlag Berlin/Heidelberg/New York/Tokyo 1983, S. 326 sowie W. Forth, D. Henschler und W. Rummel "Pharmakologie und Toxikologie", Wissenschaftsverlag Mannheim/Wien/Zürich, 5. Auflage, S. 389 - 392 und "Magnesium: Its Biologic Significance" von J. K. Aikawa, CRC Press, Inc., Boca Raton, Florida, S. 112 - 113). Daher ist die Verabreichung von Magnesium- und Eisenpräparaten, insbesondere an schwangere Frauen, angezeigt.

Aus der CH-A-294 891 sind oral verabreichbare Nährsalzpräparate bekannt, die dem menschlichen Körper Metallkomponenten zuführen sollen, an denen es ihm mangelt, wenn die Nahrung nicht richtig zusammengesetzt ist und um gleichzeitig Säuremangel im Magen zu beheben bzw. einen Säureüberschuß zu neutralisieren. Das in der CH-A-294891 angegebene Nährsalzpräparat ist nicht für spezielle Applikationen vorgesehen und enthält gemäß dem dortigen Ausführungsbeispiel jeweils 350 g Zitronensäure in 700 g Wasser, 125 g Natriumhydroxid in etwa 200 g Wasser und 8 g Kaliumchlorid, 10 g Kalziumchlorid(CaCl₂), 0,5 g Eisenchlorid(FeCl₂), 5 g Magnesiumchlorid(MgCl₂) und 0,25 g Manganchlorid, die dann zusammen in etwa 100 g Wasser aufgelöst werden, wobei man insgesamt 1 Liter Wasser verwendet und diese drei Lösungen miteinander vermischt.

Die bei sachgerechter Herstellung laut Einnahmeempfehlung zugeführten Mengen an Magnesium und Eisen sind jedoch augenfällig viel zu gering und die Zufuhr an Natrium nach den heutigen Kenntnissen bei weitem zu hoch. Die in der CH-A-294 891 vorgesehene Herstellung von 3 Lösungen, die dann zu einer einzigen Lösung kombiniert werden, ist sehr umständlich und erfordert die Mitarbeit des Patienten. Fehler bei der Herstellung der Nährsalzlösungen sind durch die komplizierte "Zusammenmischerei" nicht auszuschließen.

Die Erfindung hat sich daher die Aufgabe gestellt, unter Beseitigung der vorstehend aufgezeigten Nachteile des Standes der Technik ein oral verabreichbares pharmazeutisches Mittel zur Verfügung zu stellen, mit welchem es möglich ist, gleichzeitig Magnesium- und Eisenverbindungen ohne Verringerung der Eisenresorption zu verabreichen.

Diese Aufgabe wird erfindungsgemäß durch Anspruch 1 gelöst, und zwar durch Verwendung einer oder mehrerer pharmazeutisch und physiologisch verträglicher Eisenverbindung(en), einer oder mehrerer pharmazeutisch und physiologisch verträglicher Magnesiumverbindung(en) sowie, falls die Magnesiumverbindung(en) keine die verfügbaren Magnesiumvalenzen absättigenden, in wäßrigen Medien dissozierbaren Chloratome enthält (enthalten), einer oder mehrerer pharmazeutisch und physiologisch verträglicher, in wäßrigen Medien Chlorionen liefernden Verbindung(en) zusammen mit üblichen pharmazeutischen Lösungs- und/oder Verdünnungsmitteln und/oder Hilfsstoffen zur Herstellung eines pharmazeutischen Mittels zur Eisen- und Magnesiumsubstitutionstherapie, wobei die verabreichten Tagesdosen zwischen 5 bis 100 mg Eisen liegen.

In den Unteransprüchen sind vorteilhafte Ausgestaltungen der vorliegenden Erfindung wiedergegeben.

Die Erfindung beruht auf der Feststellung, daß überraschenderweise die gleichzeitige Verabreichung von pharmazeutischen Magnesium- und Eisenverbindungen ohne Verringerung der Bioverfügbarkeit von Eisen dann möglich ist, wenn zusammen mit den Magnesium- und Eisenverbindungen eine in wäßrigen Medien Chlorionen liefernde Verbindung in einer solchen Menge verabreicht wird, daß die im Magensaft freigesetzten exogenen Chlorionen zumindest den verfügbaren Magnesium-Valenzen der eingesetzten Magnesiumverbindungen äquivalent sind. Im Falle einer Untersäuerung des Magensafts ist es zweckmäßig, die Chlorionen liefernde Verbindung in noch größeren Mengen einzusetzen, um in der Magenflüssigkeit eine möglichst hohe physiologische Chlorionenkonzentration zu erzeugen.

Mit anderen Worten ausgedrückt ist es erfindungswesentlich, bei gleichzeitiger Verabreichung einer Magnesiumverbindung, die keine Chloratome zur Absättigung der Magnesiumvalenzen enthält, wie beispielsweise Magnesiumoxid, Magnesiumhydroxid, Magnesiumaspartat oder -glutamat, Chlorionen im Magen in einer solchen Menge zur Verfügung zu stellen, die zur Absättigung der Magnesiumvalenzen erforderlich sind, wobei es bei einer Untersäuerung der Magenflüssigkeit zweckmäßig ist, eine über diese Menge hinausgehende Chlorionenmenge bereitzustellen. Sind diese Bedingungen erfüllt, dann wird die verabreichte Eisenverbindung überraschenderweise in dem gleichen Ausmaß resorbiert wie dies dann der Fall ist, wenn keine Magnesiumverbindung gleichzeitig verabreicht wird.

Die gleichen Bedingungen gelten, wenn erfindungsgemäß zusätzlich eine oder mehrere Kalziumverbindungen, wie Kalziumsalze, beispielsweise Kalziumchlorid, Kalziumglukonat, Kalziumaspartat, zur Beseitigung von den bekannten Kalziummangelzuständen, verwendet werden. Dies bedeutet, daß dann, wenn eine chlorfreie Kalziumverbindung erfindungsgemäß in dem Mittel eingesetzt wird, durch eine Chlorionen liefernde Verbindung soviel Chlorionen zur Verfügung gestellt werden müssen, die zumindest den beiden Kalziumvalenzen äquivalent sind, d. h. pro Kalziumion zwei Chlorionen.

Die Chlorionen liefernden Verbindungen, die erforderlichenfalls in dem Mittel erfindungsgemäß eingesetzt werden, bestehen vorzugsweise aus anorganischen Chloriden, insbesondere Magnesiumchlorid, Kalziumchlorid, Eisen(II)chlorid, Eisen(III)chlorid oder Kaliumchlorid oder aus Chlorwasserstoffsäure Bei normalem pH-Gehalt des Magensafts kann die Verabreichung einer oder mehrerer Chlorionen liefernder Verbindungen dann entfallen, wenn eine Magnesiumverbindung und/oder Kalziumverbindung eingesetzt wird, in welcher die Magnesiumvalenzen bzw. Kalziumvalenzen durch Chloratome abgesättigt sind, wie beispielsweise Magnesiumchlorid oder Kalziumchlorid, wobei in besonders bevorzugter Weise als Magnesiumverbindungen Magnesiumaminodicarbonsäurechloride, insbesonder Magnesiumaspartatchlorid und Magnesiumglutamatchlorid, eingesetzt werden, die von allen bisher in der Magnesiumtherapie eingesetzten Magnesiumverbindungen weitaus am besten resorbiert werden. Diese Magnesiumaminodicarbonsäurechloride und ihr Einsatz in der Therapie sind bekannt (vgl. beispielsweise die DE-OS 32 38 118, die ein neues Verfahren zur Herstellung dieser Verbindungen beschreibt).

Als Magnesiumverbindungen, deren frei verfügbare Magnesiumvalenzen nicht durch Chloratome abgesättigt sind, können beispielsweise Magnesiumoxid, Magnesiumhydroxid, Magnesiumcarbonat, Magnesiumaspartat, Magnesiumglutamat, Magnesiumhydrogenphosphat, Magnesiumglycerophosphat, Magnesiumtrisilikat, Magnesiumhydroxidcarbonat, Magnesiumacetat, Magnesiumcitrat, Magnesiumgluconat, Magnesiumlactat, Magnesiumorotat oder Magnesiumaminodicarbonsäurefluoride, -bromide, und -iodide, die ebenfalls als Pharmazeutika bekannt sind und deren Herstellung beispielsweise in der vor stehend erwähnten DE-OS beschrieben wird, verwendet werden. In diesem Falle sind natürlich Chlorionen liefernde Verbindungen zusätzlich in dem Präparat erfindungsgemäß einzusetzen.

Als Eisenverbindungen kommen vorzugsweise Eisen(II)- und Eisen(III)salze in Frage, insbesondere die Chloride, ferner Sulfate und Gluconate.

Die pharmazeutisch verabreichbaren Mittel können in üblicher Weise durch Vermischen der Bestandteile Zusammen mit üblichen Lösungs- und Verdünnungsmitteln und/oder -trägern und Konfektionierung zu üblichen Darreichungsformen, wie Tabletten, Kapseln, Granulaten oder Lösungen hergestellt werden.

Die unter Einsatz der Mittel verabreichbaren Tagesdosen liegen zwischen 20 und 240 mg Magnesium, 5 bis 100 mg Eisen und 500 bis 1000 mg Kalzium.

Das folgende Beispiel erläutert die Erfindung:

### Beispiel

24 weibliche SD-Ratten mit einem Anfangsgewicht von ca. 130 g wurden zufallsgemäß auf 4 Gruppen (n = 6) verteilt. Während eines Zeitraums von 20 Tagen erhielten die Tiere unterschiedliche DIäten. Gruppe I war Kontrollgruppe und bekam eine Mangel-Diät in Pulverform verabreicht, die durch Zusatz von Mg-Aspartat-HCl auf 500 ppm und Fe-Gluconat auf 200 ppm angereichert wurde. Gruppe II, III und IV erhielten die Mangel-Diät ohne Mg- und Fe-Zusatz. (Ermittelte Gehalte der Mangel-Diät: Mg. 87 ppm; Fe: 5,3 ppm). Am Morgen des 21. Tages wurden die Tiere vom Futter abgesetzt. Nach 24 Stunden bekamen die Tiere folgende Lösungen via Schlundsonde verabreicht:

| | |
|---|---|
| Gruppe I | 2 ml H₂O |
| Gruppe II | 2 ml Fe-Gluconat (= 8,65 mg Fe) |
| Gruppe III | 1 ml Mg-Aspartat (= 37,45 mg Mg) |
| | 1 ml Fe-Gluconat (= 8,65 mg Fe) |
| Gruppe IV | 1 ml Mg-Aspartat-HCL (= 37,5 mg Mg) |
| | 1 ml Fe-Gluconat (= 8,65 mg Fe). |

Ein Tier aus Gruppe IV starb kurz nach der Sondierung.

Nach 3 Stunden erfolgte die Blutentnahme mittels Punktion der Aorta Abdominalis. Im Plasma wurden der Mg- und Fe-Gehalt mit AAS bestimmt. Es ergaben sich folgende Werte:

| | Fe | Mg | |
|---|---|---|---|
| Gruppe I | 3,325 ppm ± 0,65 (0,059 mmol/l) | 15,12 ppm ± 1,17 (0,62 mmol/l) | (n=6) |
| Gruppe II | 3,6 ppm ± 0,69 (0,065 mmol/l | 6,8 ppm ± 2,07 (0,25 mmol/l) | (n=6) |
| Gruppe III | 2,78 ppm ± 0,5 (0,05 mmol/l) | 20,9 ppm ± 6,4 (0,86 mmol/l) | (n=6) |
| Gruppe IV | 3,15 ppm ± 0,63 (0,056 mmol/l) | 27,33 ppm ± 5,9 (1,12 mmol/l) | (n=5) |

Statistisch wurden die Gruppenunterschiede mittels Varianzanalyse festgelegt und durch einen multible-range-Test (Duncan) auf dem 95 % Niveau geprüft. Signifikante Unterschiede ergaben sich bei dem Fe-Gehalt zwischen Gruppe II und III (p < 0,05), d.h. Mg-Aspartat verschlechtert die Fe-Resorption, wogegen Mg-Aspartat-HCl diese nicht signifikant beeinflußt.

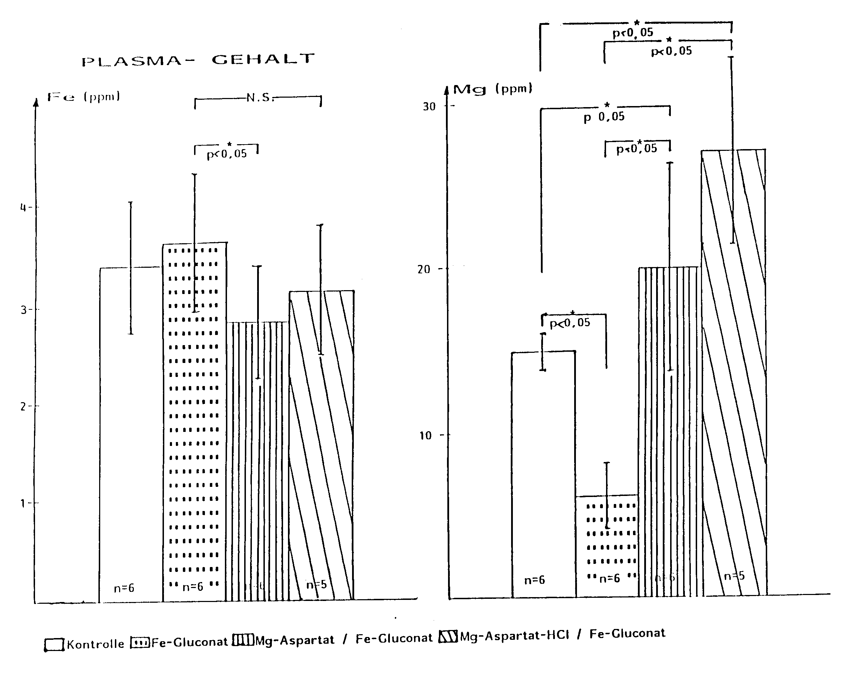

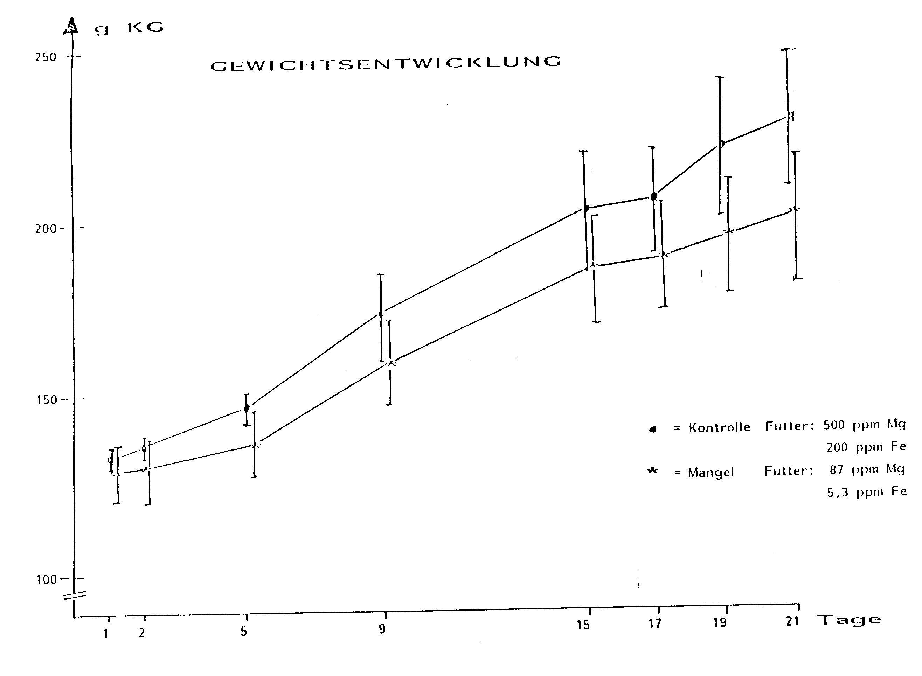

## Patentansprüche

1. Verwendung einer oder mehrerer pharmazeutisch und physiologisch verträglicher Eisenverbindung(en), einer oder mehrerer pharmazeutisch und physiologisch verträglicher Magnesiumverbindung(en) sowie, falls die Magnesiumverbindung(en) keine die verfügbaren Magnesiumvalenzen absättigenden, in wäßrigen Medien dissozierbaren Chloratome enthält (enthalten), einer oder mehrerer pharmazeutisch und physiologisch verträglicher, in wäßrigen Medien Chlorionen liefernden Verbindung(en) zusammen mit üblichen pharmazeutischen Lösungs- und/oder Verdünnungsmitteln und/oder Hilfsstoffen zur Herstellung eines pharmazeutischen Mittels zur Eisen- und Magnesiumsubstitutionstherapie, wobei die verabreichten Tagesdosen zwischen 5 bis 100 mg Eisen liegen.

2. Verwendung nach Anspruch 1, dadurch gekennzeichnet, daß das Mittel zusätzlich eine oder mehrere pharmazeutisch verträgliche Kalziumverbindung(en) enthält.

3. Verwendung nach Anspruch 1 oder 2, dadurch gekennzeichnet, daß das Mittel eine oder mehrere pharmazeutisch und physiologisch verträgliche, in wäßrigen Medien Chlorionen-liefernde Verbindung(en) enthält, die in einer solchen Menge vorliegt (vorliegen), daß Chlorionen in Magensaft in einer Menge zur Verfügung gestellt werden, die zumindest den nicht durch Chloratome abgesättigten Valenzen der Magnesiumverbindung(en) äquivalent sind.

4. Verwendung nach einem der Ansprüche 1 bis 3, dadurch gekennzeichnet, daß die Chlorionen liefernde(n) Verbindung(en) aus anorganischen Chloriden, vorzugsweise Magnesiumchlorid, Kalziumchlorid, Eisen(II)chlorid, Eisen(III)chlorid oder Kaliumchlorid oder aus Chlorwasserstoffsäure bestehen.

5. Verwendung nach einem der Ansprüche 1 bis 3, dadurch gekennzeichnet, daß das Mittel als Magnesiumverbindungen eine oder mehrere ionogene Chlor enthaltende Magnesiumverbindungen enthält.

6. Verwendung nach einem der Ansprüche 1 bis 5, dadurch gekennzeichnet, daß das Mittel als Magnesiumverbindungen, Magnesiumaspartatchlorid und Magnesiumglutamatchlorid enthält.

7. Verwendung nach einem der Ansprüche 1 bis 5, dadurchgekennzeichnet, daß das Mittel als Eisenverbindungen Eisen(II)- und/oder Eisen(III)salze, insbesondere Chloride, Sulfate und Gluconate enthält.

8. Verwendung nach einem der Ansprüche 1 bis 5, dadurch gekennzeichnet, daß das Mittel als Kalziumverbindungen Kalziumsalze, insbesondere Kalziumchlorid, Kalziumaspartathydrochlorid oder Kalziumglutamathydrochlorid enthält.

## Claims

1. Use of one or more pharmaceutically and physiologically compatible iron compounds, one or more pharmaceutically and physiologically compatible magnesium compounds and, if the magnesium compound(s) does (do) not contain any chlorine atoms which saturate the available magnesium valences and can be dissociated in aqueous media, one or more pharmaceutically and physiologically compatible compounds which in aqueous media supplies (supply) chlorine ions, together with conventional pharmaceutical solvents and/or diluents and/or auxiliaries, for the preparation of a pharmaceutical composition for iron and magnesium substitution therapy, the daily doses administered being between 5 and 100 mg of iron.

2. Use according to Claim 1, characterized in that the composition additionally contains one or more pharmaceutically compatible calcium compounds.

3. Use according to Claim 1 or 2, characterized in that the composition contains one or more pharmaceutically and physiologically compatible compounds which in aqueous media supply (supplies) chlorine ions and which is (are) present in a quantity such that chlorine ions are made available in gastric juice in a quantity which is at least equivalent to those valences of the magnesium compound(s) which are not saturated by chlorine atoms.

4. Use according to one of Claims 1 to 3, characterized in that the compound(s) which supplies (supply) chlorine ions comprise(s) inorganic chlorides, preferably magnesium chloride, calcium chloride, iron (II) chloride, iron(III) chloride or potassium chloride, or hydrochloric acid.

5. Use according to one of Claims 1 to 3, characterized in that the composition contains as magnesium compounds one or more ionogenic chlorine-containing magnesium compounds.

6. Use according to one of Claims 1 to 5, characterized in that the composition contains as magnesium compounds magnesium aspartate chloride and magnesium glutamate chloride.

7. Use according to one of Claims 1 to 5, characterized in that the composition contains as iron compounds iron (II) and/or iron (III) salts, especially chlorides, sulphates and gluconates.

8. Use according to one of Claims 1 to 5, characterized in that the composition contains as calcium compounds calcium salts, especially calcium chloride, calcium aspartate hydrochloride or calcium glutamate hydrochloride.

## Revendications

1. Utilisation d'un ou plusieurs composés du fer pharmaceutiquement et physiologiquement acceptables, d'un ou plusieurs composés du magnésium pharmaceutiquement et physiologiquement acceptables, ainsi qu'un ou plusieurs composés pharmaceutiquement et physiologiquement acceptables livrant des ions chlore en milieu aqueux, pour le cas où le ou les composés du magnésium ne contiennent pas d'atomes de chlore pouvant être dissociés en milieu aqueux et saturant les valences disponibles du magnésium, conjointement avec des solvants et/ou des diluants et/ou des adjuvants pharmaceutiques usuels, pour la préparation d'un agent pharmaceutique pour la thérapie de substitution du fer et du magnésium, les doses journalières administrées étant comprises entre 5 et 100 mg de fer.

2. Utilisation selon la revendication 1, caractérisée en ce que l'agent contient, en outre, un ou plusieurs composés du calcium pharmaceutiquement acceptables.

3. Utilisation selon la revendication 1 ou 2, caractérisée en ce que l'agent contient un ou plusieurs composés pharmaceutiquement et physiologiquement acceptables livrant des ions chlore en milieu aqueux, en une quantité telle que des ions chlore sont libérés dans le suc gastrique en une quantité qui équivaut au moins aux valences, non saturées par des atomes de chlore, du ou des composés du magnésium.

4. Utilisation selon l'une des revendications 1 à 3, caractérisée en ce que le ou les composés livrant des ions chlore sont formés de chlorures minéraux, de préférence le chlorure de magnésium, le chlorure de calcium, le chlorure de fer(II), le chlorure de fer(III) ou le chlorure de potassium, ou d'acide chlorhydrique.

5. Utilisation selon l'une des revendications 1 à 3, caractérisée en ce que l'agent contient, comme composés du magnésium, un ou plusieurs composés ionogènes du magnésium contenant du chlore.

6. Utilisation selon l'une des revendications 1 à 5, caractérisée en ce que l'agent contient, comme composés du magnésium, le chlorure aspartate de magnésium et le chlorure glutamate de magnésium.

7. Utilisation selon l'une des revendications 1 à 5, caractérisée en ce que l'agent contient, comme composés du fer, des sels de fer(II) et/ou de fer(III), en particulier les chlorures, les sulfates et les gluconates.

8. Utilisation selon l'une des revendications 1 à 5, caractérisée en ce que l'agent contient, comme composés du calcium, des sels de calcium, en particulier le chlorure de calcium, le chlorhydrate aspartate de calcium ou le chlorhydrate glutamate de calcium.
